Europäisches Patentamt

European Patent Office

Office européen des brevets

(19)

(11) Publication number: **0 109 989**
**B1**

(12) **EUROPEAN PATENT SPECIFICATION**

(45) Date of publication of patent specification: **26.03.86**

(21) Application number: **82306298.9**

(22) Date of filing: **25.11.82**

(51) Int. Cl.⁴: **C 07 C 2/88, C 07 C 11/02, C 07 F 5/06**

(54) **Linear alpha olefin production using a tank growth reactor.**

(43) Date of publication of application:
**13.06.84 Bulletin 84/24**

(45) Publication of the grant of the patent:
**26.03.86 Bulletin 86/13**

(84) Designated Contracting States:
**BE DE FR GB IT NL**

(56) References cited:
**DE-A-1 920 726**
**US-A-3 457 322**
**US-A-4 314 090**

(73) Proprietor: **THE DOW CHEMICAL COMPANY**
**2030 Dow Center Abbott Road P.O. Box 1967**
**Midland, MI 48640 (US)**

(72) Inventor: **Fowler, Allan Eugene**
**52 Forest Court**
**Lake Jackson Texas (US)**
Inventor: **White, Gordon Eldon**
**210 Nasturtium**
**Lake Jackson Texas (US)**
Inventor: **Sims, Steve Austin**
**728 Milton**
**Angleton Texas (US)**

(74) Representative: **Burford, Anthony Frederick**
**et al**
**W.H. Beck, Greener & Co. 7 Stone Buildings**
**Lincoln's Inn**
**London WC2A 3SZ (GB)**

Courier Press, Leamington Spa, England.

## Description

The production of alpha-olefins having unbranched carbon skeletal configurations and terminal unsaturation has been practiced for many years. With the advent of the triorgano-metallic compounds, viz. triethyl and tri-n-butyl aluminum, large scale commercial production has occurred. The primary and desired products are the $C_{12}$ to $C_{18}$ alpha-olefins for detergent use and the $C_{10+}$ for synthetic lubricants. The lower carbon atom compounds, viz. $C_6$ to $C_8$ have recently found utility in the polymer field and thus are being recovered in increasing volume.

Conventionally, the alpha-olefins are produced in a two stage process in which a growth reaction and subsequent displacement reaction are carried out as independent process steps. In the growth reaction, $C_2$—$C_4$ olefin or a mixture thereof is reacted with aluminum trialkyl to form higher alkyls of aluminum. These higher alkyls of aluminum are split in the displacement reaction by reaction with $C_2$—$C_4$ olefin or a mixture thereof to form the required higher alpha olefins and regenerate the aluminum trialkyl.

U.S. Patent No. 3,457,322 discloses a one-stage process in which both the growth reaction and the displacement reaction are conducted in a fractionator. The feed olefin is introduced into the lower region of the fractionator and the aluminum trialkyl is introduced into an intermediate region of the fractionator. The temperatures at the top and bottom of the fractionator are maintained as 125 to 325°F (50 to 160°C) and 350 to 600°F (180 to 320°C) respectively and the pressure in the fractionator is 100 to 10,000 psig (0.7 to 70 MPa). The aluminum trialkyl is present as 0.0001 to 0.015 mol per mole of olefin feed.

In U.S. Patent 4,314,090, there is disclosed a process which provides for the production of increased amounts of $C_4$—$C_{10}$ alpha-olefins using an elongate coil growth reactor such as well known from U.S. 2,971,969. The growth reaction is carried out independently of the displacement reaction using, in the growth reaction zone, a temperature of 100 to 400°C, a pressure of 400 to 800 psig (2.8 to 5.5 MPa), a residence time of 5 to 90 minutes and a weight ratio of feed olefin to aluminum trialkyl of 5:1 to 20:1. However, these reactors require an olefin:aluminum alkyl ratio of about 10 to 1 for best results.

It has been found that by using a tank reactor with a relatively rapid external recirculation rate, instead of the coil reactor of U.S. Patent No. 4,314,090, it is possible to use 2 to 6 moles of olefin per mole of aluminum alkyl and achieve yields of the desired hexene-1 and octene-1 after the displacement reaction which is as high as the system using coil reactors. An added advantage of the present invention is the reduction in the amount of energy and capital equipment costs required by using the lower ratios of olefin. In fact, no excess olefin is required thereby eliminating costly recycle compression.

According to the present invention, there is provided a process for making $C_4$—$C_{10}$ alpha-olefins wherein an alpha-olefin having 2—4 carbon atoms or a mixture thereof and a low molecular weight trialkyl aluminum are reacted in a growth reaction zone under growth promoting conditions to provide higher molecular weight trialkyl aluminum and subsequently reacting an alpha-olefin having 2—4 carbon atoms or a mixture thereof with said higher trialkyl aluminum in a displacement reactor zone separate from said growth reactor zone and under displacement conditions to provide a mixture of $C_4$—$C_{10}$ alpha-olefins, characterised in that the growth reaction zone is a tank reaction zone containing 2 to 6 moles of alpha-olefin per mole of trialkyl aluminum and has a recirculation rate through an external heat transfer zone such that the tank reaction zone contents are completely recirculated in a period of time sufficient to remove the heat of said reaction zone and maintain a substantially constant temperature therein.

Preferably, the growth promoting conditions used in the tank reactor are a temperature of 100 to 150°C and preferably 115 to 125°C, a pressure range from 1.5—13.9 MPa and preferably 2.2—10.5 MPa and a liquid residence time of 15 to 60 minutes and preferably 30 to 40 minutes.

The growth reaction zone contents are recirculated through an external heat transfer zone at a recirculation rate such that the reaction zone contents are completely recirculated for a time sufficient to remove the heat of the reaction and maintain a substantially constant temperature. Preferably the recirculation time period is 0.1 to 30.0 minutes and 0.5 to 3.0 minutes is the most preferred time period.

A further aspect of the present invention is the use of a displacement zone containing a static mixer having 3 to 30 fixed elements which speeds up the displacement reaction.

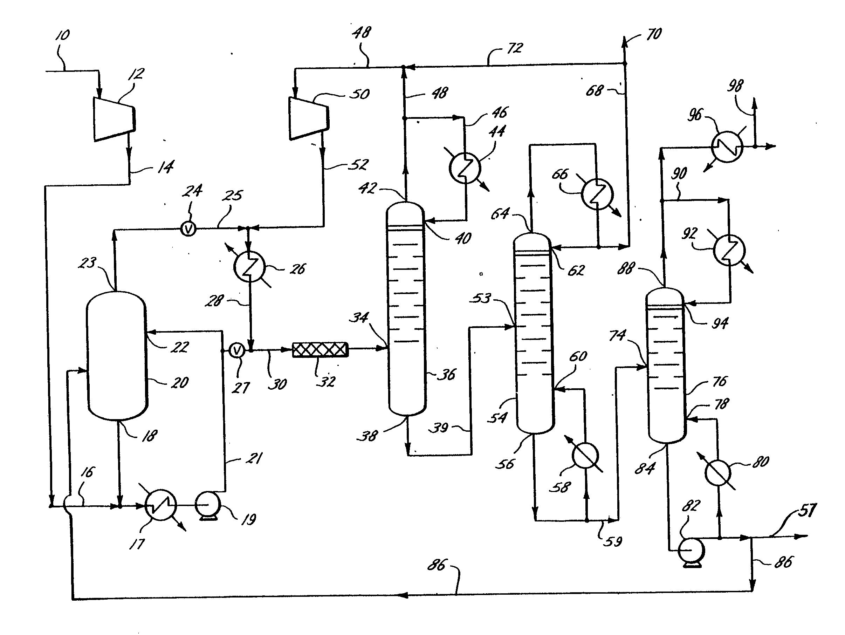

The invention is further illustrated by the Figure of the attached drawing.

In the drawing, 10 is a feed line supplying ethylene, propylene, or butene-1, and preferably ethylene which has been suitably purified to remove moisture and oxygen. Item 12 is a compressor wherein the lower olefin feed is brought up to a pressure of about 4.9 MPa. The compressed olefin is then fed by line 14 to line 16 where it is combined with recycled catalyst from line 18 and circulated through the heat exchanger 17, the pump 19 and the recycle line 21 to the reactor inlet 22.

The pressurized tank or growth reactor 20 is provided with a gaseous outlet 23 for unreacted gases which circulate via line 25 through a heat exchanger 26 and line 28 to the displacement feed line 30. The combined liquid feed from line 30 and the gas feed from line 28 are thus combined to provide a feed to the displacement reactor 32. Reduction valves 24 and 27 are provided to maintain the high pressure in the reactor 20.

In the displacement reactor 32, liquid growth material is reacted with the displacement gases to generate growth α-olefins of $C_4$—$C_{20}$ and also

recover the organo metallic catalyst. The reactor 32 is a conventional static mixer.

The contents of the displacement reactor 32 are fed by line 34 into the extraction column 36 wherein lower olefins or ethylene is stripped from the reaction mixture and is removed by outlet 42, and line 48. A recycle line 46, condenser 44 and a recycle inlet 40 is provided to remove the heavier gases and return them as liquid reflux. The line 48 carries the lower olefins to a compressor 50 where the gases are brought back up to the proper pressure for introduction by line 52 into the recycle line 28.

The bottoms from the tower 36 are removed by outlet 38 and introduced by line 39 into the tower 54 by inlet 53. In a manner similar to the tower 36, a higher olefin such as butene gas is stripped via the outlet 64, condenser 66 and inlet 62, with line 68 removing the butene gas to a takeoff valve 70 and transfer line 72. If desired, some or most of the butene can be withdrawn from valve 70 for use as an intermediate. The remaining butene is thus recycled by line 72 back to line 48 for reuse as a displacing gas.

The liquid bottoms in the butene tower 54 are recirculated via outlet 56, re-boiler 58, and inlet 60. The bottoms are fed by line 59 to inlet 74 of the vacuum tower 76 wherein the desired $\alpha$-olefins $(C_6—C_{10})$ are separated from the catalyst mixture. The vacuum tower 76 is provided with a vacuum line 98 for the necessary reduction in pressure.

The bottoms from the vacuum tower 76 are drawn off by outlet 84 and circulated by pump 82 through a re-boiler 80 and back to the tower 76 by inlet 78.

A portion of the vacuum tower bottoms are recycled by line 86 back to the growth reactor 20. A reflux circuit for the vacuum tower 76 is provided by line 90, condenser 92 and inlet 94. A purge line 57 is provided to periodically remove $C_{12}$ and higher molecular weight material.

The vacuum tower overhead products are removed by outlet 88 and sent to a heat exchanger 96 and removed for further separation into the desired $\alpha$-olefins.

In accordance with the present invention, a lower olefin such as ethylene after being pressurized to a range from 2.2—7.0 MPa is fed to a tank reactor maintained at a pressure of from 1.5—13.9 MPa and preferably from 2.2—10.5 MPa and a temperature of 100°C to 150°C and preferably from 115°C to 125°C wherein the olefin reacts with a trialkyl aluminum growth material, viz. triethyl aluminum or tributyl aluminum and preferably a mixture of the two, high in triethyl aluminum content. The reactor is of such size to enable the reactants to be in contact between 5 and 90 minutes and preferably from 30 to 40 minutes. Due to the exothermic reaction, the pressurized tank reactor is provided with an external heat transfer device so that the reaction zone contents are completely recirculated in a period of time from 0.25 to 5 minutes and preferably 0.5 to 3 minutes. Product withdrawn from the growth reactor is cooled to insure the growth products and any unreacted growth material remain in the liquid state yet the olefin or ethylene which has not reacted can be separated and returned to the growth reactor as recycle olefin.

The cooled liquid growth product and any unreacted growth material is then mixed with a displacement gas, such as $\alpha$-olefin of 2—4 carbon atoms or preferably a mixture of ethylene and butene-1 in a static mixer displacement reactor of such size as to displace the material fed in under six seconds. The displacement gas is preheated to a temperature such that when mixed with the liquid feed stream at the displacement reactor, the temperature will be in the range from 200°C to 350°C and preferably in the range from 250°C to 280°C. The reactor pressure is maintained at 0.1—7.0 MPa and preferably in the range from 0.8—1.5 MPa. The static mixture has 3 to 30 elements and preferably 6 to 12 elements so that a relatively short contact time in the range from 0.1 to 5 seconds, and is preferably in the range from 0.5 to 1.0 seconds, is achieved with complete mixing of the gas and liquid. The mol ratio of the displacement gas to each alkyl group of the trialkyl aluminum is in the range from 10:1 to 50:1 and preferably in the range 25:1 to 30:1.

The displacement product, the olefins and the tri lower alkyl aluminum product, is rapidly cooled to below about 150°C and fed to the first stage of a separator 36 wherein the displacement gas and the olefins are removed and the remaining liquid is sent to a second stage separator 54 and the liquid product remaining is directed to a third stage separator 76. The gaseous products delivered to the second stage separator are further cooled and the displacement gases, removed, recompressed, heated, and recycled to the displacement reactor feed stream. The liquid bottoms product from the second separation is the desired olefins, in this case $C_4$ to $C_{10}$ $\alpha$-olefins, which may be further separated in its constituent components. The third separator strips any remaining $\alpha$-olefins from the tri lower alkyl aluminum growth material which olefins are combined with the $\alpha$-olefins from the second separator and the latter, the lower alkyl aluminum compounds are recycled to the growth reactor.

Illustrative of the effectiveness of the · tank reaction zone with recirculation, the conditions and results of thirteen typical examples are shown below wherein all percentages are in weight percent.

Example 1
A 250 cc tank reactor was charged with the following materials:

| | |
|---|---|
| triethyl aluminum (TEA) | 17.07 gms |
| tri-n-butyl aluminum (TNBA) | 11.38 gms |
| tetradecane (n-$C_{14}$) | 28.45 gms |

This represents a 50% by weight aluminum alkyl feed stream.

The reactor had a bottom outlet connected to a recycle pump which pumped the reactor contents through a cooling coil back into the reactor.

A total of 31.8 grams of ethylene gas was charged at 4.8 MPa. The reactor was brought to a 126°C operating temperature via electric tape heaters. The pump was started and set to flow 50 cc/min. of liquid. This represents a 1 1/2 minute reactor volume turnover.

The reaction was allowed to run for 30 minutes and then cooled down to room temperature. The aluminum alkyls were collected and a material balance was done. The aluminum alkyls, when slowly hydrolyzed, showed the following olefin yields:

| | |
|---|---|
| Butene-1 | 22.0% wt. |
| Hexene-1 | 58.7% wt. |
| Octene-1 | 16.6% wt. |
| Decene-1 | 2.6% wt. |
| $C_{12+}$ | 0.1% wt. |
| Total | 100.0% wt. |

Example 2

Using the same reactor set up as Example No. 1, the following conditions were noted:

Charge

| | |
|---|---|
| TEA | 11.4 gms |
| TNBA | 7.6 gms |
| $n-C_{14}$ | 76.0 gms |

The solution recycle rate was set at 50 cc/min. with the reactor operating at 4.9 MPa and 113°C. A total of 35.3 grams of ethylene was charged to the reactor during the reactor operation.

After 30 minutes residence time, the following yields were noted:

| | |
|---|---|
| Butene-1 | 8.9% wt. |
| Hexene-1 | 61.3% wt. |
| Octene-1 | 23.7% wt. |
| Decene-1 | 5.8% wt. |
| $C_{12+}$ | 0.3% wt. |

Example 3

Using the same reactor set up as Example No. 1, the following conditions were noted:

Charge

| | |
|---|---|
| triethyl aluminum (TEA) | 28.5 grams |
| tri n-butyl aluminum (TNBA) | 19.0 grams |
| tetradecane ($n-C_{14}$) | 47.5 grams |

The solution recycle rate was set at 50 cc/min. This represents a 2.5 minute reactor volume turnover. A total 27.8 grams ethylene gas was charged with the reactor operating at 4.9 MPa and 117°C for 20 minutes residence time.

The following yields were noted:

| | |
|---|---|
| Butene-1 | 18.9% wt. |
| Hexene-1 | 66.9% wt. |
| Octene-1 | 13.0% wt. |
| $C_{12+}$ | 0.0% wt. |

Example 4

Using the same reactor set up as Example No. 1, the following conditions were noted:

Charge

| | |
|---|---|
| TEA | 16.9 grams |
| TNBA | 11.3 grams |
| $n-C_{14}$ | 28.2 grams |

The solution recycle rate was set at 75 cc/min. This represents a 1.0 minute reactor volume turnover. A total 32.2 grams ethylene gas was charged with the reactor operating at 4.9 MPa and 120°C for 30 minutes residence time.

The following yields were noted:

| | |
|---|---|
| Butene-1 | 19.6% wt. |
| Hexene-1 | 57.4% wt. |
| Octene-1 | 18.5% wt. |
| Decene-1 | 4.0% wt. |
| $C_{12+}$ | 0.5% wt. |

Example 5

Using the same reactor set up as Example No. 1, the following conditions were noted:

Charge

| | |
|---|---|
| TEA | 17.0 grams |
| TNBA | 11.3 grams |
| $n-C_{14}$ | 28.4 grams |

The solution recycle rate was set at 50 cc/min. This represents a 1.5 minute reactor volume turnover. A total 19.8 grams ethylene gas was charged with the reactor operating at 3.6 MPa and 120°C for 30 minutes residence time.

The following yields were noted:

| | |
|---|---|
| Butene-1 | 22.2% wt. |
| Hexene-1 | 57.6% wt. |
| Octene-1 | 16.2% wt. |
| Decene-1 | 3.5% wt. |
| $C_{12+}$ | 0.5% wt. |

Example 6

Using the same reactor set up as Example No. 1, the following conditions were noted:

Charge

| | |
|---|---|
| TEA | 16.8 grams |
| TNBA | 11.2 grams |
| n-$C_{14}$ | 28.0 grams |

This solution recycle rate was set at 50 cc/min. This represents a 1.5 minute reactor volume turnover. A total 29.8 grams ethylene gas was charged with the reactor operating at 4.9 MPa and 110°C for 30 minutes residence time.

The following yields were noted:

| | |
|---|---|
| Butene-1 | 22.23% wt. |
| Hexene-1 | 60.11% wt. |
| Octene-1 | 15.0% wt. |
| Decene-1 | 2.34% wt. |
| $C_{12+}$ | 0.32% wt. |

Example 7

Using the same reactor set up as Example No. 1, the following conditions were noted:

Charge

| | |
|---|---|
| TEA | 5.7 grams |
| TNBA | 3.8 grams |
| n-$C_{14}$ | 85.3 grams |

The solution recycle rate was set at 50 cc/min. This represents a 2.5 minute reactor volume turnover. A total 31.5 grams ethylene gas was charged with the reactor operating at 4.9 MPa and 115°C for 30 minutes residence time.

The following yields were noted:

| | |
|---|---|
| Butene-1 | 16.9% wt. |
| Hexene-1 | 60.8% wt. |
| Octene-1 | 17.6% wt. |
| Decene-1 | 3.9% wt. |
| $C_{12+}$ | 0.8% wt. |

Example 8

Using the same reactor set up as Example No. 1, the following conditions were noted:

Charge

| | |
|---|---|
| TEA | 11.4 grams |
| TNBA | 7.6 grams |
| n-$C_{14}$ | 76.0 grams |

The solution recycle rate was set at 50 cc/min. This represents a 2.5 minute reactor volume turnover. A total 35.3 grams ethylene gas was charged with the reactor operating at 4.9 MPa and 113°C for 30 minutes residence time.

The following yields were noted:

| | |
|---|---|
| Butene-1 | 8.8% wt. |
| Hexene-1. | 61.3% wt. |
| Octene-1 | 23.6% wt. |
| Decene-1 | 5.8% wt. |
| $C_{12+}$ | 0.5% wt. |

Example 9

Using the same reactor set up as Example No. 1, the following conditions were noted;

Charge

| | |
|---|---|
| TEA | 17.0 grams |
| TNBA | 11.3 grams |
| n-$C_{14}$ | 28.4 grams |

The solution recycle rate was set at 50 cc/min. This represents a 1.5 minute reactor volume turnover. A total 39.0 grams ethylene gas was charged with the reactor operating at 6.3 MPa and 121°C for 30 minutes residence time.

The following yields were noted:

| | |
|---|---|
| Butene-1 | 4.3% wt. |
| Hexene-1 | 56.2% wt. |
| Octene-1 | 26.7% wt. |
| Decene-1 | 9.8% wt. |
| $C_{12+}$ | 3.0% wt. |

Example 10

Using the same reactor set up as Example No. 1, the following conditions were noted:

| Charge | |
| --- | --- |
| TEA | 22.68 grams |
| TNBA | 15.12 grams |
| n-$C_{14}$ | 37.80 grams |

The solution recycle rate was set at 67 cc/min. This represents a 1.5 minute reactor volume turnover. A total of 45.3 grams ethylene gas was charged with the reactor operating at 4.9 MPa and 130°C for 15 minutes residence time.

The following yields were noted:

| Butene-1 | 24.2% wt. |
| --- | --- |
| Hexene-1 | 55.3% wt. |
| Octene-1 | 16.4% wt. |
| Decene-1 | 3.5% wt. |
| $C_{12+}$ | 0.6% wt. |

Example 11

Using the same reactor set up as Example No. 1, the following conditions were noted:

| Charge | |
| --- | --- |
| TEA | 22.68 grams |
| TNBA | 15.12 grams |
| n-$C_{14}$ | 37.80 grams |

The solution recycle rate was set at 67 cc/min. This represents a 1.5 minute reactor volume turnover. A total of 45.3 grams ethylene gas was charged with the reactor operating at 4.9 MPa and 130°C for 30 minutes residence time.

The following yields were noted:

| Butene-1 | 21.2% wt. |
| --- | --- |
| Hexene-1 | 53.2% wt. |
| Octene-1 | 19.8% wt. |
| Decene-1 | 5.0% wt. |
| $C_{12+}$ | 0.9% wt. |

Example 12

Using the same reactor set up as Example No. 1, the following conditions were noted:

| Charge | |
| --- | --- |
| TEA | 17.04 grams |
| TNBA | 11.36 grams |
| n-$C_{14}$ | 28.40 grams |

The solution recycle rate was set at 50 cc/min. This represents a 1.5 minute reactor volume turnover. A total of 12.5 grams ethylene gas was charged with the reactor operating at 2.2 MPa and 120°C for 30 minutes residence time.

The following yields were noted:

| Butene-1 | 19.7% wt. |
| --- | --- |
| Hexene-1 | 75.4% wt. |
| Octene-1 | 4.9% wt. |
| Decene-1 | 0.0% wt. |
| $C_{12+}$ | 0.0% wt. |

Example 13

Using the same reactor set up as Example No. 1, the following conditions were noted:

| Charge | |
| --- | --- |
| TEA | 17.07 grams |
| TNBA | 11.38 grams |
| n-$C_{14}$ | 28.45 grams |

The solution recycle rate was set at 50 cc/min. This represents a 1.5 minute reactor volume turnover. A total of 67.2 grams ethylene gas was charged with the reactor operating at 10.5 MPa and 120°C for 30 minutes residence time.

The following yields were noted:

| Butene-1 | 13.3% wt. |
| --- | --- |
| Hexene-1 | 58.14% wt. |
| Octene-1 | 22.43% wt. |
| Decene-1 | 5.4% wt. |
| $C_{12+}$ | 0.73% wt. |

**Claims**

1. A process for making $C_4$—$C_{10}$ alpha-olefins wherein an alpha-olefin having 2—4 carbon atoms or a mixture thereof and a low molecular weight trialkyl aluminum are reacted in a growth reaction zone under growth promoting conditions to provide higher molecular weight trialkyl aluminum and subsequently reacting an alpha-olefin having 2—4 carbon atoms or a mixture thereof with said higher trialkyl aluminum in a displacement reactor zone separate from said growth reactor zone and under displacement conditions to provide a mixture of $C_4$—$C_{10}$ alpha-olefins, characterised in that the growth reaction zone is a tank reaction zone containing 2 to 6 moles of alpha-olefin per mole of trialkyl aluminum and has a recirculation rate through an external heat transfer zone such that the tank reaction zone contents are completely recirculated in a period of time sufficient to remove the heat of said reaction zone and maintain a substantially constant temperature therein.

2. A process as claimed in Claim 1, wherein the recirculation time is 0.1 to 30.0 minutes.

3. A process as claimed in Claim 2, wherein the recirculation time is 0.5 to 3.0 minutes.

4. A process as claimed in any one of the preceding claims, wherein the growth promoting conditions are a temperature of 100 to 150°C, a pressure of 1.5 to 13.9 MPa, and a liquid residence time from 15 to 60 minutes.

5. A process as claimed in Claim 4, wherein the growth promoting conditions are a temperature of 115 to 125°C, a pressure of 2.2 to 10.5 MPa, and a liquid residence time from 30 to 40 minutes.

6. A process as claimed in any one of the preceding claims, wherein the displacement zone has a temperature in the range from 200° to 350°C, a pressure in the range from 0.1 to 7.0 MPa and a contact time in the range from 0.1 to 5 seconds.

7. A process as claimed in Claim 6, wherein the displacement zone is under a pressure of 0.8 to 1.5 MPa and has a temperature in the range from 250° to 280°C.

8. A process as claimed in any one of the preceding claims, wherein the alpha-olefin feed to the tank reaction zone is ethylene and ethylene or butene-1 or a mixture thereof is reacted with said growth product in the displacement zone.

9. A process as claimed in any one of preceding claim, wherein said displacement zone comprises a static mixer zone.

10. A process as claimed in Claim 9, wherein said static mixer zone has 3 to 30 fixed elements.

## Patentansprüche

1. Verfahren zur Herstellung von $C_4$—$C_{10}$-alpha-Olefinen, bei dem ein alpha-Olefin mit 2 bis 4 Kohlenstoffatomen oder eine Mischung davon und ein Aluminiumtrialkyl niedrigen Molekulargewichts in einer Kettenfortpflanzungsreaktionszone unter die Kettenfortpflanzung fördernden Bedingungen zur Reaktion gebracht werden, um Aluminiumtrialkyl mit höherem Molekulargewicht zu ergeben, und nachfolgend ein alpha-Olefin mit 2 bis 4 Kohlenstoffatomen oder eine Mischung davon mit dem höheren Aluminiumtrialkyl in einer von der Kettenfortpflanzungsreaktionszone getrennten Verdrängungsreaktionszone unter Verdrängungsbedingungen reagieren gelassen wird, um eine Mischung von $C_4$—$C_{10}$-alpha-Olefinen zu ergeben, dadurch gekennzeichnet, daß die Kettenfortpflanzungsreaktionszone eine Tank-Reaktionszone ist, die 2 bis 6 Mol alpha-Olefin pro Mol Aluminiumtrialkyl enthält und eine solche Rezirkulationsgeschwindigkeit über eine äußere Wärmeaustauschzone hat, daß der Inhalt der Tank-Reaktionszone während einer Zeitspanne vollständig rezirkuliert wird, die ausreicht, um die Wärme der Reaktionszone abzuführen und eine im wesentlichen konstante Temperatur darin aufrechtzuerhalten.

2. Verfahren nach Anspruch 1, dadurch gekennzeichnet, daß die Rezirkulationszeit 0,1 bis 30,0 Minuten beträgt.

3. Verfahren nach Anspruch 2, dadurch gekennzeichnet, daß die Rezirkulationszeit 0,5 bis 3,0 Minuten beträgt.

4. Verfahren nach einem der vorhergehenden Ansprüche, dadurch gekennzeichnet, daß die die Kettenfortpflanzung fördernden Bedingungen eine Temperatur von 100 bis 150°C, ein Druck von 1,5 bis 13,9 MPa, und eine Flüssigkeits-Verweilzeit von 15 bis 60 Minuten sind.

5. Verfahren nach Anspruch 4, dadurch gekennzeichnet, daß die die Ketennfortpflanzung fördernden Bedingungen eine Temperatur von 115 bis 125°C, ein Druck von 2,2 bis 10,5 MPa und eine Flüssigkeits-Verweilzeit von 30 bis 40 Minuten sind.

6. Verfahren nach einem der vorhergehenden Ansprüche, dadurch gekennzeichnet, daß die Verdrängsszone eine Temperatur im Bereich von 200 bis 350°C, einen Druck im Bereich von 0,1 bis 7,0 MPa und eine Kontaktzeit im Bereich von 0,1 bis 5 Sekunden besitzt.

7. Verfahren nach Anspruch 6, dadurch gekennzeichnet, daß die Verdrängungszone unter einem Druck von 0,3 bis 1,5 MPa steht und eine Temperatur im Bereich von 250 bis 280°C besitzt.

8. Verfahren nach einem der vorhergehenden Ansprüche, dadurch gekennzeichnet, daß das in die Tank-Reaktionszone eingespeiste alpha-Olefin Äthylen ist und Äthylen oder Buten-1 oder eine Mischung davon mit dem Kettenfortpflanzungsprodukt in der Verdrängungszone reagieren gelassen wird.

9. Verfahren nach einem der vorhergehenden Ansprüche, dadurch gekennzeichnet, daß die Verdrängungszone eine statische Mischerzone umfaßt.

10. Verfahren nach Anspruch 9, dadurch gekennzeichnet, daß die statische Mischerzone 3 bis 30 feststehende Elemente aufweist.

## Revendications

1. Procédé de préparation d'alpha-oléfines $C_4$—$C_{10}$ dans lequel on fait réagir une alpha-oléfine ayant 2—4 atomes de carbone ou un de leurs mélanges avec un trialkyl aluminium de faible poids moléculaire dans une zône de réaction de croissance dans des conditions favorisant la croissance pour donner un trialkyl aluminium de poids moléculaire supérieur et en faisant réagir ensuite une alpha-oléfine ayant 2—4 atomes de carbone ou un de leurs mélanges avec ce trialkyl aluminium de poids moléculaire supérieur dans une zône de réacteur de déplacement séparée de ladite zne de réacteur de croissance et dans des conditions de déplacement pour fournir un mélange d'alpha-oléfines $C_4$—$C_{10}$, caractérisé en ce que la zône de réaction de croissance est une zône de réaction de réservoir contenant de 2 à 6 moles d'alpha-oléfine par mole de trialkyl aluminium et a une vitesse de recyclage à travers une zône extérieure de transfert thermique tel que la contenu de la zône de réaction de réservoir subit un recyclage complet dans une période de temps suffisante pour éliminer la chaleur de cetté zône de réaction et pour y maintenir sensiblement consante la température.

2. Procédé selon la revendication 1, dans, lequel le temps de recyclage est de 0,1 à 30,0 minutes.

3. Procédé selon la revendication 2, dans lequel le temps de recyclage est de 0,5 à 3,0 minutes.

4. Procédé selon une quelconque des revendications précédentes, dans lequel les conditions favorisant la croissance sont une température de 100 à 150°C, une pression de 1,5 à 13,9 MPa, et un temps de séjour de liquide de 15 à 60 minutes.

5. Procédé selon la revendication 4, dans lequel les conditions favorisant la croissance sont une température de 115 à 125°C, une pression de 2,2 à 10,5 MPa, et un temps de séjour du liquide de 30 à 40 minutes.

6. Procédé selon une quelconque des revendications précédentes, dans lequel la zône de déplacement a une température dans la plage de 200° à 350°C, une pression dans la plage de 0,1 à 7,0 MPa et un temps de contact dans la plage de 0,1 à 5 secondes.

7. Procédé selon la revendication 6 dans lequel la zône de déplacement est sous une pression de 0,8 à 1,5 MPa et a une température dans la plage de 250° à 280°C.

8. Procédé selon une quelconque des revendications précédentes, dans lequel J'alimentation en alpha-oléfine dans la zône de réaction de réservoir est de l'éthylène et dans lequel on fait réagir l'éthylène ou du butène-1 ou un de leur mélange avec le produit de croissance dans la zône de déplacement.

9. Procédé selon une quelconque des revendications précédentes, dans lequel la zône de déplacement comporte une zône de mélangeur statique.

10. Procédé selon la revendication 9 dans lequel la zône de mélangeur statique a de 3 à 30 éléments fixes.